# EUROPEAN PATENT SPECIFICATION

(11) **EP 3 010 930 B1**
(45) Date of publication and mention of the grant of the patent: **18.09.2019**
(21) Application number: 14742356.0
(22) Date of filing: 19.06.2014
(51) Int. Cl.: C07K 1/04

(54) **PEPTIDE-RESIN CONJUGATE AND USE THEREOF**
PEPTID-HARZ-KONJUGAT UND VERWENDUNG DAVON
CONJUGUÉ PEPTIDE-RÉSINE ET SON UTILISATION

(30) Priority: 19.06.2013 GB 201310921
(43) Date of publication of application: 27.04.2016
(73) Proprietor: Chemical & Biopharmaceutical Laboratories of Patras S.A., 26000 Patras (GR)
(72) Inventor: BARLOS, Kleomenis, GR-26000 Patras (GR); BARLOS, Kostas, GR-26000 Patras (GR); GATOS, Dimitrios, GR-26000 Patras (GR)
(74) Representative: D Young & Co LLP
(86) International application number: PCT/IB2014/062430
(87) International publication number: WO 2014/203193

(56) References cited:
- EP-A1- 2 537 856
- WO-A1-2008/040536
- WO-A2-2013/098802
- KR-A- 20080 033 120
- BERNHARDT A ET AL: "THE SOLID-PHASE SYNTHESIS OF SIDE-CHAIN-PHOSPHORYLATED PEPTIDE-4-NITROANILIDES", JOURNAL OF PEPTIDE RESEARCH, BLACKWELL PUBLISHING LTD, OXFORD; GB, vol. 50, no. 2, 1 August 1997 (1997-08-01) , pages 143-152, XP000659212, ISSN: 1397-002X
- MEIENHOFER J ET AL: "SOLID-PHASE SYNTHESIS WITH ATTACHMENT OF PEPTIDE TO RESIN THROUGH AN AMINO ACID SIDE CHAIN: not 8-LYSINE 3/4 -VASOPRESSIN", PROCEEDINGS OF THE NATIONAL ACADEMY OF SCIENCES, NATIONAL ACADEMY OF SCIENCES, vol. 68, no. 5, 1 May 1971 (1971-05-01), pages 1006-1009, XP001007913, ISSN: 0027-8424, DOI: 10.1073/PNAS.68.5.1006

## Description

The present invention relates to peptide-resin conjugates suitable for use in the synthesis of peptides. More specifically, the invention relates to short peptides containing diamino acids in their sequence and their use in the synthesis of peptide amides.

### BACKGROUND TO THE INVENTION

The use of acid labile trityl-type resins in the solid phase synthesis of peptides and protected peptides is well known in the art (see, for example, Barlos K, Chatzi O, Gatos D, Stavropoulos G., Int J Pept Protein Res. 1991 Jun;37(6):513-20). Peptide resin conjugates are typically linked to the resin via a carboxy-terminally conjugated ester linkage (see, for example, US 7,939,629). Peptide-resin conjugates linked to the resin via the side chain of a terminal lysine amino acid residue are also known (see, for example, US2009/0292106).

The commonly used Rinck-amide resins result in peptide amides, which contain in many cases several byproducts, which have their origin in the partial cleavage of the linker.

Giraud et al in US2010/0197891 describe a method of anchoring a growing peptide chain during chemical synthesis to a solid phase support via the amino group of an amino acid side chain. However, US2010/0197891 does not disclose the attachment of a peptide amide to the resin.

The present invention seeks to provide new peptide-resin conjugates for use in the synthesis of peptides. More particularly, in one embodiment, the invention seeks to provide new peptide-resin conjugates and methods relating thereto that enable the preparation of peptides exhibiting one or more of the following: improved yields, higher purity, fewer side reactions and milder reaction conditions.

Aspects of the invention are set forth in the attached claims and are described in more detail hereinbelow.

### STATEMENT OF INVENTION

A first aspect of the invention relates to a method for preparing a peptide, or a salt thereof, from a peptide-resin conjugate of Formula (2b), (2c), (2d), (2e) or (2f), 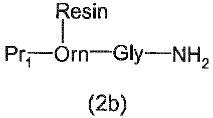 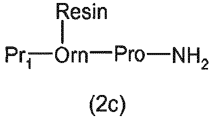 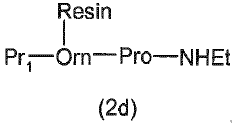 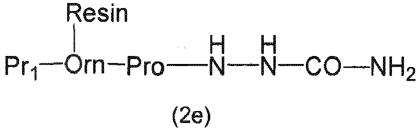 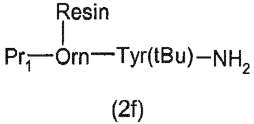
wherein the Resin is a TFA cleavable polymer resin selected from trityl, 2-chloro-trityl, 4-methyl-trityl and 4-methoxy-trityl resins; and
Pr₁ is a protecting group selected from the group consisting of Fmoc, Boc, Cbz, Npys and Alloc;
said method comprising the steps of:
   (a) deprotecting the N-terminal α-amino function of said peptide-resin conjugate of Formula (2b), (2c), (2d), (2e) or (2f) to remove protecting group Pr₁;
   (b) coupling an at least N-terminally protected amino acid or peptide having a free or activated carboxylic acid function with the deprotected α-amino function of step (a), thereby elongating the compound of formula (2b), (2c), (2d), (2e) or (2f),
   (c) optionally repeating steps (a) and (b) one or more times, wherein the at least N-terminally protected amino acid or peptide is identical or different to that of the preceding step (b);
   (d) cleaving the resulting peptide from the Resin;
   (e) guanylating the peptide obtained in step (d);
   (f) optionally removing all protecting groups which remain after step (d);
   (g) isolating and optionally purifying the peptide thus obtained.

Advantageously, using the presently claimed peptide amide conjugates allows the preparation of peptides in better yields and/or in higher purity and/or with fewer side reactions. Moreover, the mild reaction conditions required to remove the peptide amides from the resin allows the peptide amides to be obtained in protected or partially protected form, i.e. the method allows the cleavage from the resin of partially protected peptide amides. This enables the obtained peptides to be selectively converted to the corresponding guanylated peptides. The presently claimed conjugates are particularly useful in the preparation of a number of specific peptides described herein.

### DETAILED DESCRIPTION

As used herein, the term "alkyl" includes both saturated straight chain and branched alkyl groups which may be substituted (mono- or poly-) or unsubstituted. Preferably, the alkyl group is a C₁₋₂₀ alkyl group, more preferably a C₁₋₁₅, more preferably still a C₁₋₁₂ alkyl group, more preferably still, a C₁₋₆ alkyl group, more preferably a C₁₋₃ alkyl group. Particularly preferred alkyl groups include, for example, methyl, ethyl, propyl, isopropyl, butyl, isobutyl, tert-butyl, pentyl and hexyl. Suitable substituents include, for example, one or more groups selected from OH, O-alkyl, halogen, NH₂, NH-alkyl, N-(alkyl)₂, CF₃, NO₂, CN, COO-alkyl, COOH, CONH₂, CO-NH-alkyl, CO-N(alkyl)₂, SO₂-alkyl, SO₂NH₂ and SO₂-NH-alkyl.

As used herein, the term "aryl" refers to a C₆₋₁₂ aromatic group which may be substituted (mono- or poly-) or unsubstituted. Typical examples include phenyl and naphthyl etc. Suitable substituents include, for example, one or more groups selected from OH, O-alkyl, halogen, NH₂, NH-alkyl, N-(alkyl)₂, CF₃, NO₂, CN, COO-alkyl, COOH, CONH₂, CO-NH-alkyl, CO-N(alkyl)₂, SO₂-alkyl, SO₂NH₂ and SO₂-NH-alkyl.

The term "aralkyl" is used as a conjunction of the terms alkyl and aryl as given above. As used herein, the term "aroyl" refers to a radical "Ar-CO", where Ar is an aryl group as defined above. Examples of aroyl groups include benzoyl and napthoyl.

As used herein, the term "acyl" refers to a radical "alkyl-CO", where alkyl is as defined above.

Pharmaceutically acceptable salts of the compounds of the invention include suitable acid addition or base salts thereof. A review of suitable pharmaceutical salts may be found in Berge et al, J Pharm Sci, 66, 1-19 (1977). Salts are formed, for example with strong inorganic acids such as mineral acids, e.g. sulphuric acid, phosphoric acid or hydrohalic acids; with strong organic carboxylic acids, such as alkanecarboxylic acids of 1 to 4 carbon atoms which are unsubstituted or substituted (e.g., by halogen), such as acetic acid; with saturated or unsaturated dicarboxylic acids, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C₁-C₄)-alkyl- or aryl-sulfonic acids which are unsubstituted or substituted (for example, by a halogen) such as methane- or p-toluene sulfonic acid. Acetate salts are particularly preferred.

In all aspects of the present invention previously discussed, the invention includes, where appropriate all enantiomers and tautomers of the compounds of the invention. The man skilled in the art will recognise compounds that possess an optical properties (one or more chiral carbon atoms) or tautomeric characteristics. The corresponding enantiomers and/or tautomers may be isolated/prepared by methods known in the art.

Some of the compounds of the invention may exist as stereoisomers and/or geometric isomers - e.g. they may possess one or more asymmetric and/or geometric centres and so may exist in two or more stereoisomeric and/or geometric forms. The present invention contemplates the use of all the individual stereoisomers and geometric isomers of those compounds, and mixtures thereof. The terms used in the claims encompass these forms.

The present invention also includes all suitable isotopic variations of the compounds or pharmaceutically acceptable salts thereof. An isotopic variation of an agent of the present invention or a pharmaceutically acceptable salt thereof is defined as one in which at least one atom is replaced by an atom having the same atomic number but an atomic mass different from the atomic mass usually found in nature. Examples of isotopes that can be incorporated into the agent and pharmaceutically acceptable salts thereof include isotopes of hydrogen, carbon, nitrogen, oxygen, phosphorus, sulphur, fluorine and chlorine such as ²H, ³H, ¹³C, ¹⁴C, ¹⁵N, ¹⁷O, ¹⁸O, ³¹P, ³²P, ³⁵S, ¹⁸F and ³⁶Cl, respectively. Certain isotopic variations of the agent and pharmaceutically acceptable salts thereof, for example, those in which a radioactive isotope such as ³H or ¹⁴C is incorporated, are useful in drug and/or substrate tissue distribution studies. Tritiated, i.e., ³H, and carbon-14, i.e., ¹⁴C, isotopes are particularly preferred for their ease of preparation and detectability. Further, substitution with isotopes such as deuterium, i.e., ²H, may afford certain therapeutic advantages resulting from greater metabolic stability, for example, increased *in vivo* half-life or reduced dosage requirements and hence may be preferred in some circumstances. Isotopic variations of the agent of the present invention and pharmaceutically acceptable salts thereof of this invention can generally be prepared by conventional procedures using appropriate isotopic variations of suitable reagents.

Natural amino acids include alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, and valine.

As used herein, the term "non-natural amino acid" includes alpha and alpha-disubstituted amino acids, N-alkyl amino acids, lactic acid, halide derivatives of natural amino acids such as trifluorotyrosine, p-Cl-phenylalanine, p-F-phenylalanine, p-Br-phenylalanine, p-NO₂-phenylalanine, phenylglycine, azaglycine, sarcosine, penicillamine, D-2-methyltryptophan, phosphoserine, phosphothreonine, phosphotyrosine, p-I-phenylalanine, L-allyl-glycine, β-alanine, β-aspartic acid, β-cyclohexylalanine, citrulline, homoserine, homocysteine, pyroglutamic acid, L-α-amino butyric acid, L-γ-amino butyric acid, L-α-amino isobutyric acid, α-cyclohexylglycine, diaminobutyric acid, diaminopimelic acid, N-ε-dinitrophenyl-lysine, L-1-naphthylalanine, L-2-naphthylalanine, 3-(2-pyridyl)-L-alanine, 3-(3-pyridyl)-L-alanine, 3-(4-pyridyl)-L-alanine, N-ε-methyl-lysine, N,N-ε-dimethyl-lysine, N,N,N-ε-trimethyl-lysine, 3-mercaptopropionic acid, L-ε-amino caproic acid, 7-amino heptanoic acid, 6-amino hexanoic acid L-methionine sulfone, ornithine, L-norleucine, L-norvaline, p-nitro-L-phenylalanine, L-hydroxyproline, γ-glutamic acid, γ-amino butyric acid L-thioproline, methyl derivatives of phenylalanine (Phe) such as 4-methyl-Phe, pentamethyl-Phe, L-Phe (4-amino), L-Tyr (methyl), L-Phe (4-isopropyl), L-Tic (1,2,3,4-tetrahydroisoquinoline-3-carboxyl acid), L-diaminopropionic acid and L-Phe (4-benzyl).

The peptide of the present invention may comprise amino acids in the L or D form, i.e. one or more residues, preferably all the residues may be in the L or D form.

As used herein, the term "synthetic peptide" refers to a peptide that is chemically synthesized. Synthetic peptides may be prepared from natural or unnatural amino acids, or a combination thereof.

As used herein, the term "natural peptide" refers to a peptide that is found in nature.

The present inventors have demonstrated that diamino acids containing short peptide amides attached to suitable highly acid sensitive resins from their side-chain can provide larger peptide amides and partially protected peptide amides with selectively liberated diamino acid side chain amino function. These can be further selectively modified on the liberated diamino acid side chain. An important modification of the diamino acid side chain is, for example the selective guanidylation of Orn to Arg to give Arg-containing peptides. This is advantageous over the synthesis of Arg-containing peptides by using side chain protected Arg-derivatives because the Arg side chain deprotection using the usual guanidino protecting groups typically gives rise to the formation of several by-products and in addition is in many cases incomplete.

Several side products are also formed during the removal of peptide amides from resins which provide peptide amides such as the Rink-amide resins.

The present inventors have discovered that the attachment of short diamino acid-containing peptides, on resins of the trityl type, proceeds with high yield and that the peptides and partially protected peptide amides obtained are of high purity, higher than obtained by using the corresponding peptide amide synthesis method which utilizes an amide resin. The peptide amides and partially protected peptide amides cleaved from the resin can be transformed in high yield to peptides which contain in their sequence guanylated amino acids such as Arg, D-Arg, homo-Arg. The obtained peptide amides are of a higher purity than the corresponding Arg- containing peptides synthesized using side chain protected Arg-derivatives, for example, Fmoc-Arg(Pbf)-OH.

The required suitable short peptide amides (1) are obtained by methods known in the art e.g. by deprotecting the side chain amino function of the short peptide. The short protected peptide amides are subsequently conjugated with very acid sensitive resins of the trityl type through the side chain amino function of the diamino acid contained in the peptide chain according to the scheme below.

For example, the diamino acid which is contained in the C-terminal part of the peptide amide is reacted with a resin halide, A-CI, in the presence of a base to give the peptide-resin conjugate (2) in high yield: 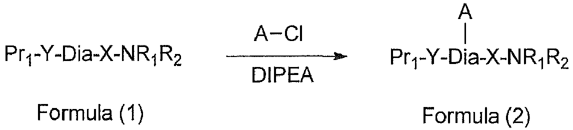
wherein Pr₁ is selected from H, alkyl, aryl, aralkyl, acyl, aroyl and a protecting group;
Y is a direct bond; or an optionally protected natural or unnatural amino acid residue; or a natural or synthetic peptide comprising 2 to 200 natural or unnatural amino acid residues, each of which is optionally protected;
Dia is a natural or unnatural diamino acid;
A is a polymer resin conjugated to the side chain amino function of the diamino acid;
X is an optionally protected natural or unnatural amino acid residue; or a peptide comprising 2 to 15 natural or unnatural amino acid residues, each of which is optionally protected;
R₁ and R₂ are each independently selected from H, alkyl, aryl, aralkyl, NH₂, NH-CO-NH₂.

In one preferred embodiment, the base is a trialkylamine base, more preferably, DIPEA.

In one preferred embodiment, A is a TFA-cleavable polymer resin conjugated on the side chain amino function of the Dia.

More preferably, A is a TFA-cleavable resin of the trityl type.

Even more preferably, A is selected from trityl, 2-chloro-trityl, 4-methyl-trityl and 4-methoxy-trityl resins as shown below, wherein Q can be absent, or is a linker between the trityl-group and the polymer matrix P, such as a carboxyl group.

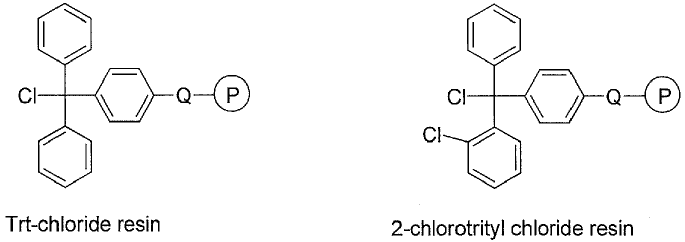

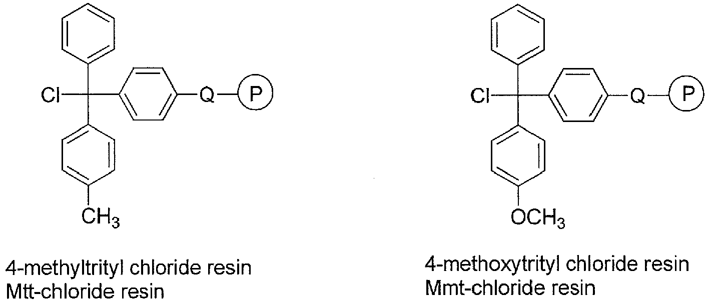

Resin-bound peptide amides can also be obtained by attachment of the diamino acid to the resin through the side chain amino function and coupling on resin with the amino acid amide or peptide amide as shown in the scheme below:

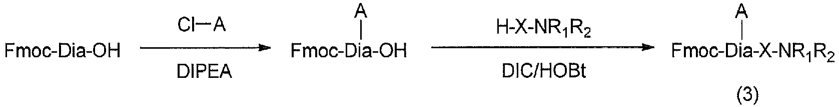

Peptide amides (3) can also be obtained by amidating resin-bound peptides as shown below:

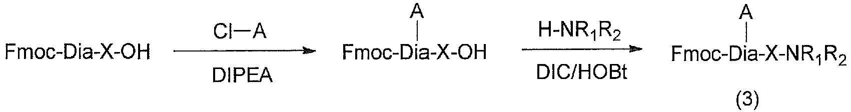

The resin-bound peptides of formula (2) and (3) can be used in solid phase peptide synthesis. After completion of the chain assembly, the obtained peptide amides of the general formula (2) can be cleaved from the extremely acid sensitive resins in the partially protected peptide form of the general formula (1) or in the complete deprotected form as shown in the scheme below with the general formula (4):

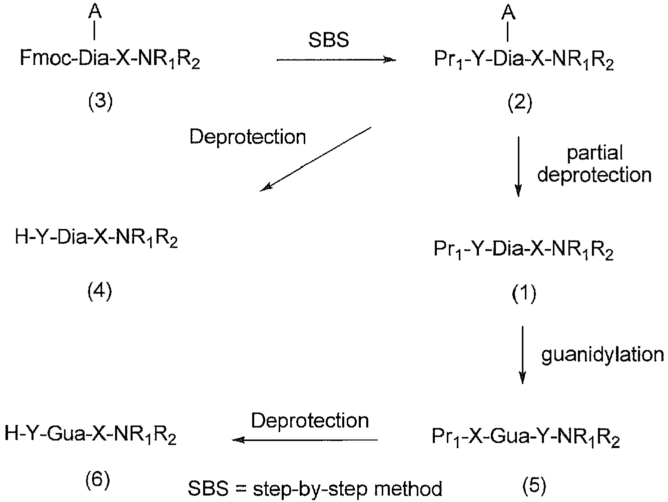

The selectively at the diamino acid side chain function deprotected peptides (1) can be further modified at the side chain of the diamino acid as shown in the scheme above by guanidylation. The guanidylation step can be performed by any method known in the art for, example, using guanidylation reagents such as S-methylthiourea, 1-H-1,2,4-triazole-carboxamidine etc.

In one preferred embodiment, the guanidylation reagent is selected from 1H-pyrazole-1-carboxamidine 2, 1-H-1,2,4-triazole-carboxamidines, triflyl guanidine and benzotriazole-1-carboxamidinium tosylate.

The obtained guanidylated partially protected peptides of the general formula (5) can then be totally deprotected to give guanidyl side chain function containing peptides of the general formula (6), wherein Gua is a side chain guanidyl-group containing amino acid such as Arg.

Suitable protecting groups for amino acids will be familiar to the person skilled in the art (see for example, Chem. Rev. 2009, 109, 2455-2504). These protecting groups can be separated into three groups, as follows:
- N-terminal protecting groups
- C-terminal protecting groups
- side chain protecting groups

Examples of highly preferred N-terminal protecting groups for amino acids include, but are not limited to, t-Boc (*tert*-butyloxycarbonyl) and Fmoc (9-fluorenylmethyloxycarbonyl). Their lability is caused by the carbamate group which readily releases CO₂ for an irreversible decoupling step. Another suitable carbamate based group is the benzyloxy-carbonyl (Z or Cbz) group; this is removed in harsher conditions.

Another preferred example is the allyloxycarbonyl (alloc) protecting group, which is often used to protect a carboxylic acid, hydroxyl, or amino group when an orthogonal deprotection scheme is required.

Another preferred example is the nitro-2-pyridinesulfenyl (Npys) group, which is useful for the protection and activation of amino and hydroxyl groups in peptide synthesis. The Npys group is readily introduced by treatment of amino acids with 3-nitro-2-pyridinesulfenyl chloride. The Npys group is easily removed by treatment with very dilute HCI, e.g. 0.1-0.2 N HCI in dioxane, but is is resistant to trifluoroacetic acid and 88% formic acid. Npys is also selectively removed under neutral conditions using triphenylphosphine or 2-pyridinethiol 1-oxide without affecting benzyloxycarbonyl (Z), tert-butyloxycarbonyl (Boc), 2-(4-biphenylyl)propyl(2)oxycarbonyl (Bpoc), 9-fluorenylmethyloxycarbonyl (Fmoc), benzyl (Bzl) or tert-butyl (tBu) protecting groups
Amino acid side chains represent a broad range of functional groups and are sites of nonspecific reactivity during peptide synthesis. Because of this, many different protecting groups are required that are usually based on the benzyl (Bzl) or tert-butyl (tBu) group. The specific protecting groups used during the synthesis of a given peptide vary depending on the peptide sequence and the type of N-terminal protection used. Side chain protecting groups are generally known as permanent or semi-permanent protecting groups, because they can withstand the multiple cycles of chemical treatment during synthesis and are only removed during treatment with strong acids after peptide synthesis is completed.

Purified, individual amino acids are reacted with these protecting groups prior to synthesis and then selectively removed during specific steps of peptide synthesis.

In one preferred embodiment, Pr₁ is an Fmoc protecting group.

More preferably, the Resin is trityl or 4-methoxy-trityl resin.

The peptide resin conjugates can be prepared by a process which comprises reacting a peptide amide with a suitable resin halide in a suitable solvent in the presence of a base.

Preferably, the halide is selected from chloride, bromide and iodide.

Preferably, the solvent is selected from DCM, DCE, DMF, NMP, THF, DME and mixtures thereof.

Preferably, the base is selected from DIPEA, NMM, DBU, pyridine, DMAP and TEA.

In one preferred embodiment, the N-terminally protected amino acids or peptides of steps (b) and (c) are Fmoc-protected.

In one preferred embodiment, the at least N-terminally protected amino acid or peptide of the lastly repeated step (c) is protected by an protecting group which is orthogonal to Fmoc.

In one preferred embodiment, the orthogonal protecting group is Boc.

In one preferred embodiment, step (d) comprises cleaving the peptide from the resin by treatment with an acid.

In one preferred embodiment, step (e) comprises treating the peptide with a guanylating reagent selected from 1H-pyrazole-1-carboxamidine 2, 1-H-1,2,4-triazole-carboxamidine, triflyl guanidine and benzotriazole-1-carboxamidinium tosylate.

In one preferred embodiment, the peptide resin-conjugate is selected from the following: 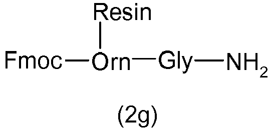 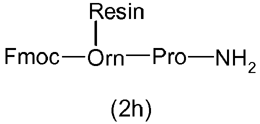 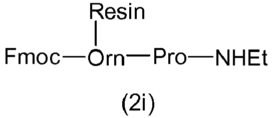 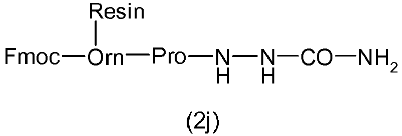 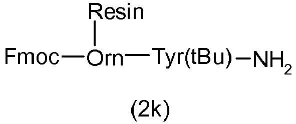 wherein the Resin is trityl resin or 4-methoxytrityl resin.

In one preferred embodiment, the method of the invention is used for preparing a peptide selected from the following:
[2] Arg⁸-Cys-Tyr-Phe-Gln-Asn-Cys-Pro-Arg-Gly-NH₂;
[5] 3-Mercaptopropionyl-Tyr-Phe-Gln-Asn-Cys-Pro-D-Arg-Gly-NH₂;
[8] Pyr-His-Trp-Ser-Tyr-D-Ser(tBu)-Leu-Arg-Pro-Azagly-NH₂;
[9] Pyr-His-Trp-Ser-Tyr-D-Ser(tBu)-Leu-Arg-Pro-NHEt acetate salt;
[10] Pyr-His-Trp-Ser-Tyr-D-Leu-Leu-Arg-Pro-NHEt;
[11] Pyr-His-Trp-Ser-Tyr-D-His(Bzl)-Leu-Arg-Pro-NHEt acetate salt;
[12] Pyr-His-Trp-Ser-Tyr-D-Trp-Leu-Arg-Pro-NHEt acetate salt;
[20] 
H-Tyr-Pro-Ser-Lys-Pro-Asp-Asn-Pro-Gly-Glu-Asp-Ala-Pro-Ala-Glu-Asp-Met-
Ala-Arg-Tyr-Tyr-Ser-Ala-Leu-Arg-His-Tyr-Ile-Asn-Leu-Ile-Thr-Arg-Gln-Arg-Tyr-
NH2; and
[21]

H-Tyr-Pro-Ile-Lys-Pro-Glu-Ala-Pro-Gly-Glu-Asp-Ala-Ser-Pro-Glu-Glu-Leu-Asn-
Arg-Tyr-Tyr-Ala-Ser-Leu-Arg-His-Tyr-Leu-Asn-Leu-Val-Thr-Arg-Gln-Arg-Tyr-
NH2.

Specific peptides prepared by the method of the invention include the following:
* denotes outside the scope of invention
***Atosiban acetate,**
   3-Mercaptopropionyl-D-Tyr(Et)-Ile-Thr-Asn-Cys-Pro-Orn-Gly-NH₂ acetate salt
**Vasopressin acetate,**
   Arg-Cys-Tyr-Phe-Gln-Asn-Cys-Pro-Arg-Gly-NH₂ acetate salt
***Ornipressin acetate,**
   H-Cys-Tyr-Phe-Gln-Asn-Cys-Pro-Orn-Gly-NH₂ acetate salt
***Terlipressin acetate,**
   H-Gly-Gly-Gly-Cys-Tyr-Phe-Gln-Asn-Cys-Pro-Lys-Gly-NH₂ acetate salt
**Desmopressin acetate,**
   3-Mercaptopropionyl-Tyr-Phe-Gln-Asn-Cys-Pro-D-Arg-Gly-NH₂ acetate salt
***Triptorelin acetate,**
   Pyr-His-Trp-Ser-Tyr-D-Trp-Leu-Arg-Pro-Gly-NH₂ acetate salt
***Nafarelin Acetate**
   Pyr-His-Trp-Ser-Tyr-D-2-Nal-Leu-Arg-Pro-Gly-NH₂ acetate salt
**Goserelin**
   Pyr-His-Trp-Ser-Tyr-D-Ser(tBu)-Leu-Arg-Pro-Azagly-NH₂
**Buserelin acetate,**
   Pyr-His-Trp-Ser-Tyr-D-Ser(tBu)-Leu-Arg-Pro-NHEt acetate salt
**Leuprolide (Leuprolin)**
   Pyr-His-Trp-Ser-Tyr-D-Leu-Leu-Arg-Pro-NHEt
**Histrelin Acetate**
   Pyr-His-Trp-Ser-Tyr-D-His(Bzl)-Leu-Arg-Pro-NHEt acetate salt
**Deslorelin High Acetate**
   Pyr-His-Trp-Ser-Tyr-D-Trp-Leu-Arg-Pro-NHEt acetate salt
***Cetrorelix** 
Ac-D-2-Nal-4-chloro-D-Phe-β-(3-pyridyl)-D-Ala-Ser-Tyr-D-Cit-Leu-Arg-Pro-D-Ala-NH2
acetate salt
***Ozarelix** 
Ac-D-2-Nal-D-4-Cpa-D-3-Pal-Ser-N-Me-Tyr-D-Hci-Nle-Arg-Pro-D-Ala-NH2
acetate salt
***Ziconotide acetate** 
H-Cys-Lys-Gly-Lys-Gly-Ala-Lys-Cys-Ser-Arg-Leu-Met-Tyr-Asp-Cys-Cys-Thr-Gly-Ser-
Cys-Arg-Ser-Gly-Lys-Cys-NH2 acetate salt
***Afamelanotide**
   Ac-Ser-Tyr-Ser-Nle-Glu-His-D-Phe-Arg-Trp-Gly-Lys-Pro-Val-NH₂ acetate salt
***GRF (human) Acetate** 
H-Tyr-Ala-Asp-Ala-Ile-Phe-Thr-Asn-Ser-Tyr-Arg-Lys-Val-Leu-Gly-Gln-Leu-Ser-Ala-Arg-
Lys-Leu-Leu-Gln-Asp-Ile-Met-Ser-Arg-Gln-Gln-Gly-Glu-Ser-Asn-Gln-Glu-Arg-Gly-Ala-
Arg-Ala-Arg-Leu-NH2 acetate salt
***GRF (1-29) amide (human)** 
H-Tyr-Ala-Asp-Ala-Ile-Phe-Thr-Asn-Ser-Tyr-Arg-Lys-Val-Leu-Gly-Gln-Leu-Ser-Ala-Arg-
Lys-Leu-Leu-Gln-Asp-Ile-Met-Ser-Arg-NH2
***α-Melanotropin (human) Acetate**
   Ac-Ser-Tyr-Ser-Met-Glu-His-Phe-Arg-Trp-Gly-Lys-Pro-Val-NH₂ acetate salt
**Neuropeptide Y (human, rat) Acetate** 
H-Tyr-Pro-Ser-Lys-Pro-Asp-Asn-Pro-Gly-Glu-Asp-Ala-Pro-Ala-Glu-Asp-Met-Ala-Arg-
Tyr-Tyr-Ser-Ala-Leu-Arg-His-Tyr-Ile-Asn-Leu-Ile-Thr-Arg-Gln-Arg-Tyr-NH2 acetate salt
**Peptide YY (human) Acetate**

H-Tyr-Pro-Ile-Lys-Pro-Glu-Ala-Pro-Gly-Glu-Asp-Ala-Ser-Pro-Glu-Glu-Leu-Asn-Arg-Tyr-
Tyr-Ala-Ser-Leu-Arg-His-Tyr-Leu-Asn-Leu-Val-Thr-Arg-Gln-Arg-Tyr-NH2 acetate salt

The method of the invention is suitable for preparing a variety of different peptides including but not limited to the following:
[2] Arg-Cys-Tyr-Phe-Gln-Asn-Cys-Pro-Arg-Gly-NH₂;
[5] 3-Mercaptopropionyl-Tyr-Phe-Gln-Asn-Cys-Pro-D-Arg-Gly-NH₂ acetate salt;
[8] Pyr-His-Trp-Ser-Tyr-D-Ser(tBu)-Leu-Arg-Pro-Azagly-NH₂;
[9] Pyr-His-Trp-Ser-Tyr-D-Ser(tBu)-Leu-Arg-Pro-NHEt acetate salt;
[10] Pyr-His-Trp-Ser-Tyr-D-Leu-Leu-Arg-Pro-NHEt;
[11] Pyr-His-Trp-Ser-Tyr-D-His(Bzl)-Leu-Arg-Pro-NHEt acetate salt;
[12] Pyr-His-Trp-Ser-Tyr-D-Trp-Leu-Arg-Pro-NHEt acetate salt;
[20] 
H-Tyr-Pro-Ser-Lys-Pro-Asp-Asn-Pro-Gly-Glu-Asp-Ala-Pro-Ala-Glu-Asp-Met-
Ala-Arg-Tyr-Tyr-Ser-Ala-Leu-Arg-His-Tyr-Ile-Asn-Leu-Ile-Thr-Arg-Gln-Arg-Tyr-
NH2 acetate salt; and
[21]

H-Tyr-Pro-Ile-Lys-Pro-Glu-Ala-Pro-Gly-Glu-Asp-Ala-Ser-Pro-Glu-Glu-Leu-Asn-
Arg-Tyr-Tyr-Ala-Ser-Leu-Arg-His-Tyr-Leu-Asn-Leu-Val-Thr-Arg-Gln-Arg-Tyr-
NH2 acetate salt.

Advantageously, the presently claimed method allows peptide amides and partially protected peptide amides cleaved from the resin to be transformed in high yield to peptides which contain in their sequence guanylated amino acids such as Arg, D-Arg, homo-Arg. The obtained peptide amides are of a higher purity than the corresponding Arg- containing peptides synthesized using side chain protected Arg-derivatives, for example, Fmoc-Arg(Pbf)-OH.

The present invention is further described by way of the following non-limiting examples.

### EXAMPLES

### Solid-phase synthesis of peptides and of their protected segments.

### General procedure.

### Preparation of trityl resins loaded with peptides attached through the side chain of a diamino acid, general procedure

Trityl chloride resin (100 g; loading 0.9-1.6 mmol/g) of CBL-Patras, was placed in a 2 L peptide synthesis reactor and swelled with 700 mL dichloromethane (DCM) for 30 min at 25°C. The resin was filtered and a solution of 100 mmol Fmoc-peptide acid and diisopropylethylamine (DIEA) in DCM was added so that the mmol ratio of Fmoc-peptide/DIPEA become 0,80. The mixture was shacked under nitrogen for 4 hours at 25°C. Then, the remaining active sites of the resin were neutralised by adding 10 mL of methanol (MeOH) and reacting for 1 hour at RT. The resin was filtered and washed 4X with 400 mL DMF, deswelled with 3 washes of 500 mL isopropanol (IPA) and 4X 400 ml DEE. The resin was dried to constant weight. 60-80% of the mmol of the used peptide was bound on the resin.

All of the trityl type resins described in the patent are commercially available (CBL-Patras and others).

### A3. Amidation of peptide acids attached on solid-phase through an amino acid side chain of a diamino acid, general procedure.

A resin-bound peptide (5.0 g = 0.2-1.2 mmol/g) was placed in a solid-phase reactor and treated with 0.25-1.4 mmol HOBt and DIC. The mixture was shacked for 1 h at RT and then a solution of ammonia or Alkylamine in DMF or HOBt.NH₃ salt or of a peptide amide in DMF were added in a 1.00-1.5 molar excess. The mixture was then shacked for 2 h at RT and the procedure was repeated once. The resin is then filtered and washed 5X DMF, 3X IPA and 3X DEE and dried in vacuum to constant weight.

### B. Solid-phase synthesis, a general protocol

### B1. Swelling of the resin

The resin was placed in a 15 ml reactor and treated twice with 7 mL NMP, followed by filtration.

### B2. Activation of the amino acid

The amino acid (3.0 equiv.) and 1-hydroxybenzotriazol (4.0 equiv.) was weighted and dissolved in a reactor with 2.5 their volume in NMP and cooled to 0°C. DIC was then added (3.0 equiv.) and the mixture was stirred for 15 min.

### B3. Coupling

The solution which was prepared in B2 was then added to the B1 reactor. The reactor was washed once with one volume of DCM and was added to the reactor which was stirred for 1-3 h at 25°-30°C. In a sample the Kaiser Test was performed to determine the completion of the reaction. If the coupling reaction was not completed after 3 h (positive Kaiser Test), the reaction mixture was filtered and recoupled with a fresh solution of activated amino acid. After completion of the coupling the reaction mixture was filtered and washed 4 times with NMP (5 volumes per wash).

### B4. Removal of the Fmoc-group

The resulting resin in B3 was filtered and then treated for 30 min with 5 mL of a solution which contained 25% by volume of piperidine. The resin is then washed three times with 5 mL NMP.

### B5. Elongation of the peptide chain

After the incorporation of each amino acid the steps B1-B5 were repeated until the completion of the peptide chain.

For the introduction of each individual amino acid the following Fmoc-amino acids were used: Fmoc-Gly-OH, Fmoc-Ala-OH, Fmoc-Val-OH, Fmoc-Ile-OH, Fmoc-Leu-OH, Fmoc-Met-OH, Fmoc-Phe-OH, Fmoc-Pro-OH, Fmoc-Asp(tBu)-OH, Fmoc-Glu(tBu)-OH, Fmoc-Lys(Boc)-OH, Fmoc-Orn(Mtt)-OH, Fmoc-Orn(Mmt)-OH, Fmoc-Orn(Boc)-OH, Fmoc-Lys(Mmt)-OH, Fmoc-Lys(Mtt)-OH, Fmoc-Ser(tBu)-OH, Fmoc-Ser(Trt)-OH, Fmoc-Thr(tBu)-OH, Fmoc-Thr(Trt)-OH, Fmoc-Tyr(tBu)-OH, Fmoc-Tyr(Clt)-OH, Fmoc-Asn-OH, Fmoc-Asn(Trt)-OH, Fmoc-Gln-OH, Fmoc-Gln(Trt)-OH, Fmoc-Arg(Pbf)-OH, Fmoc-His(Trt)-OH, Fmoc-Cys(Trt)-OH, Fmoc-Cys(Mmt)-OH and Fmoc-Cys(Acm)-OH and the following Boc-amino acids: Boc-Phe-OH, and Boc-Gly-OH.

### C. General method for the cleavage from resins of the trityl-type of partially protected peptides and of their protected segments which contain Fmoc- or Boc-groups or an other suitable protecting group on their N-terminus and are selectively deprotected at an individual lysine, ornithine or any other diamino acid side chain.

The resin-bound peptide or peptide segment which was produced as described above in B1-B5 and was attached at a specific Lys, Orn, or any other diamino acid side chain with a 2-chlorotrityl (Clt), Trt- Mmt or Mtt-resin was washed 4 times with 5 mL NMP, 3 times with 5 ml IPA and finally 5 times with 7 ml DCM to remove completely any residual NMP or other basic components. The resin was then cooled to 0°C, filtered from DCM and was treated six times with a solution of 10 mL 1.0-1.5% TFA in DCM/TES(95:5) at 5°C. The resin was filtered and washed three times with 10 mL DCM. Pyridine is then added to the filtrates (1.3 equiv. relative to TFA) to neutralize the TFA. The cleavage solution in DCM was then mixed with an equal volume of water.

The resulting mixture was distilled at reduced pressure to remove DCM (350 torr at 28°C). The peptide or peptide segment precipitated after the removal of DCM. The resulting peptide was washed with water and ether and dried at 30-35°C under 15 Torr vacuum. Alternatively DCM can be removed in vacuum and the partially protected peptide can be precipitate by the addition of DEE or diisopropyl ether (DIE).

### D. General method for the guanylation of the partially protected peptide which is selectively deprotected at the side chain of one or more diamino acids.

The partially at the diamino acid side chain deprotected peptide (1.0 mmol) is dissolved in 10-15 ml DMF or an appropriate mixture of DMF/water and the solution is then neutralized by the addition of DIPEA or 1N-NaOH. Then a 1.0-1.5 molar excess of the guanylation reagent e.g. of 1-H-1,2,4-triazole-carboxamidine hydrochloride is added and the mixture stirred until the completion of the guanylation is determined by HPLC, TLC or the Kaiser test. The mixture is then diluted with brine and the product is extracted in the organic phase with EtAc or DCM followed by a standard acid/base extraction. The obtained solution of the guanylated peptide is then concentrated in the RE precipitated with the addition of DEE or DIE and deprotected according to our general method below.

### E. Peptide deprotection - General method.

The partially protected peptide obtained as described above (0.01-0.005 mmol) was treated with 10 mL TFA/TES or TIPS/thioanisol/water (85:5:5:5) or TFA/DTT/water (90:5:5 for 3 h at 5°C and for 1 h at 15°C. The resulting solution was concentrated in vacuum and then the deprotected peptide was precipitated by the addition of DEE or DIE and washed three times with 10 mL DEE or DIE. The resulting solid was dried in vacuum (25°C, 1-10 Torr) until constant weight.

### Example 1. Synthesis of Atosiban starting from Fmoc-Orn-Gly-NH2 attached through the side chain of ornithine on the trityl resin.

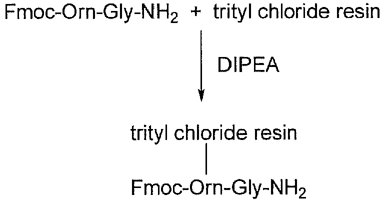

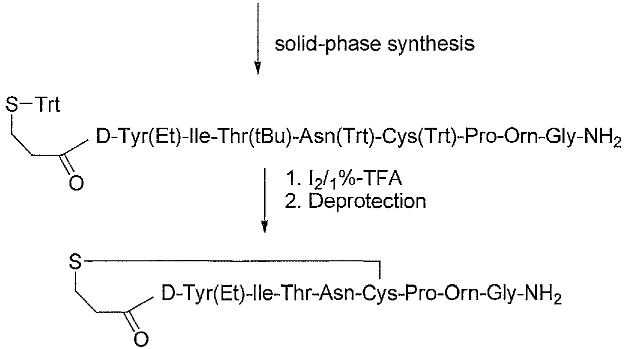

Trityl chloride resin (20.0 g; loading 24.4 mmol) of trityl chloride resin was placed in a peptide synthesis reactor and swelled with 200 mL DCM/DMF (1:1) for 30 min at 25°C. Then 4.63 g (10 mmol) of Fmoc-Orn-Gly-NH₂.HCl -produced by procedures known in the art from Fmoc-Orn(Boc)-Gly-NH₂ and HCI in dioxane- were added. The mixture was shacked over night at RT. Then, the remaining active sites of the resin were neutralised by adding 10 mL of methanol (MeOH) and reacting for additional 2 h at RT. The resin was then filtered and washed 4X with 400 mL DMF, deswelled with 3 washes of 500 mL isopropanol (IPA) and 4X 400 ml DEE and swelled again in DMF. Then, the peptide chain elongation was performed according to the standard procedures using Fmoc-Pro-OH, Fmoc-Cys(Trt)-OH, Fmoc-Asn(Trt)-OH, Fmoc-Thr(Trt)-OH, Fmoc-Ile-OH and Fmoc-D-Tyr(Et)-OH. The N-terminal S-Trt-mercapto propionic acid (MPA) was introduced finally by a similar procedure used for the introduction of the Fmoc-amino acids. The resin was then washed 6X with DMF and 6X with DCM. Then the resin was treated 6X with 100 ml of 1% TFA in DCM and to the combined filtrates 2.54 g (100 mmol) of iodine were added and the solution was stirred until the completion of the oxidation reaction (20 min). Then the excess iodine was neutralized by extraction with a 3%-Na₂S₂O₃ solution and the DCM was removed in the RE and precipitated with DEE. The obtained peptide was then deprotected using standard procedures, purified by HPLC and lyophilized. Yield: 7.14 g TFA salt, 78% peptide content (44.8%).

### Example 2. Synthesis of Atosiban starting from Fmoc-Orn-OH attached through the side chain of ornithine on the 4-methoxy resin.

1 mmol of Fmoc-Orn-OH was dissolved in 15 ml DCM. Then, 1.5 mmol of DIPEA was added and 1 g 4-methoxytrityl resin (1.2 mmol/g) and the mixture stirred overnight. 1 ml methanol was added and the mixture was stirred for an additional 4 hours at RT. The resin was then filtered, washed 3 x DCM, 3 x DMF, 3 x iPrOH and 3 x hexane and dried in vacuum to constant weight to give Fmoc-Orn(4-methoxytrityl resin)-OH.

2.0 g (1.0 mmol) of Fmoc-Orn(4-methoxytrityl resin)-OH were suspended in 10.0 ml DMF, cooled to 5°C and reacted with 0.18 g (1.3 mmol) HOBt and 0.13 g (1.0 mmol) DIC. The mixture was shacked for 5 min at 5°C and then warmed up to 15°C. Then 0.22 g (2 mmol) glycine amide hydrochloride and 0.39 g (3.0 mmol) DIPEA were added and the mixture was shacked for 90 min at RT. The procedure was repeated and the obtained Fmoc-Orn(4-methoxytrityl resin)-Gly-NH₂ was used for the synthesis of Atosiban as described above. Yield 0.79 g TFA salt of 76% peptide content (48.6 %).

### Example 3. Synthesis of resin-bound Fmoc-Orn-Pro-NH₂, Fmoc-Orn-Pro-NHEt and Fmoc-Orn-Pro-NH-NH-CO-NH₂, general procedure.

Fmoc-Orn(Boc)-OH or Fmoc-Orn(Mtt)-OH or Fmoc-Orn(Mmt)-OH were coupled by methods known in the art with H-Pro-NH₂ or H-Pro-NHEt or H-Pro-NH-NH-CO-NH₂. The obtained product was then side chain deprotected with TFA in DCM. The obtained well dried ornithine dipeptide 10.0 mmol was then dissolved in 100 ml DCM/DMF (1:1). This solution was then added to 10.0 g of 2-chlorotrityl- or trityl-, or 4-methyltrityl- or 4 methoxytrityl-chloride resin (loading = 0.9-1.6 mmol/g) and to the obtained mixture 30,0 mmol DIPEA were added. The mixture was then shacked for 12 h at RT and then 5,0 ml MeOH were added and the mixture was shacked for additional 2 h at RT. The resin was then filtered and washed 6X with DMF, 3X IPA and 3X DEE, and dried in vacuum to constant weight. Yield 12.5-14, 5 g with a total loading of 7.9-8.7 mmol.

### Example 4. Synthesis of Fmoc-Orn(4-methoxytrityl resin)-Tyr(tBu)-NH₂

Fmoc-Orn(Mtt)-OH or Fmoc-Orn(Mmt)-OH were coupled by methods known in the art with H-Tyr(tBu)-NH₂ The obtained product was then side chain deprotected with 1%-TFA in DCM/TES (97:3) for 2 h at RT. The obtained well dried ornithine dipeptide 1.0 mmol was then dissolved in 10 ml DCM/DMF (1:1). This solution was then added to 2.0 g of 4-methoxytrityl-chloride resin (loading = 1.42 mmol/g) and to the obtained mixture 3,0 mmol DIPEA were added. The mixture was then shacked for 12 h at RT and then 0.5 ml MeOH were added and the mixture was shacked for additional 2 h at RT. The resin was then filtered and washed 6X with DMF, 3X IPA and 3X hexane, and dried in vacuum to constant weight. Yield 3.02 g with a total loading of 0.86 mmol (0.28 mmol dipeptide/g).

### Example 5. Synthesis of Pyr-His-Trp-Ser-Tyr-D-Ser(tBu)-Leu-Arg-Pro-Azagly-NH₂ (Goserelin).

1.0 g (0.64 mmol) of Fmoc-Orn(4-methoxytrityl resin)-Pro-NH-NH-CO-NH2 were coupled sequentially with Fmoc-Leu-OH, Fmoc-D-Ser(tBu)-OH, Fmoc-Tyr(Clt)-OH, Fmoc-Ser(Trt)-OH, Fmoc-Trp-OH, Fmoc-His(Mmt)-OH and pyroglutamic acid. The obtained resin was then washed 6XDMF, 3XIPA and 3XDEE and dried in vacuum. Then 20 ml of 1%-TFA in DCM/TES (97:3) were added and the resin was filtered and washed with 1%-TFA in DCM/TES (97:3). The combined filtrates were then concentrated in vacuum, precipitated by the addition of DEE and dried in air. The remaining resin was then washed 3X DMF and 4X DMF/water (1:1). To the combined filtrates the solid obtained by the treating of the resin with TFA was added and the solution was neutralized by the addition of 1N-NaOH. Then 145.5 mg (1.0 mmol) 1-H-1,2,4-triazole-carboxamidine hydrochloride and 258 mg (2,0 mmol) DIPEA were added and the mixture was stirred overnight. The mixture was then acidified to pH=2.5, concentrated and purified by RP-HPLC and lyophilized. Yield: 555.3 mg (67.6%).

### Example 6. Synthesis of Pyr-His-Trp-Ser-Tyr-D-Ser(tBu)-Leu-Arg-Pro-NHEt (Buserelin)

1.0 g (0.55 mmol) of Fmoc-Orn-Pro-NHEt were coupled sequentially with Fmoc-Leu-OH, Fmoc-D-Ser(tBu)-OH, Fmoc-Tyr(Clt)-OH, Fmoc-Ser(Trt)-OH, Fmoc-Trp-OH, Fmoc-His(Mmt)-OH and pyroglutamic acid. According to the procedure described above we obtained 477.0 mg peptide (61.8%).

### Example 7. Synthesis of Pyr-His-Trp-Ser-Tyr-D-Leu-Leu-Arg-Pro-NHEt (Leuprolide, Leuprolin)

1.0 g (0,55 mmol) of Fmoc-Orn-Pro-NHEt were coupled sequentially with Fmoc-Leu-OH, Fmoc-D-Leu-OH, Fmoc-Tyr(Clt)-OH, Fmoc-Ser(Trt)-OH, Fmoc-Trp-OH, Fmoc-His(Mmt)-OH and pyroglutamic acid. According to the procedure described above we obtained 488,.1 mg (61.5%).

### Example 8. Synthesis of H-Tyr-Pro-Ile-Lys-Pro-Glu-Ala-Pro-Gly-Glu-Asp-Ala-Ser-Pro-Glu-Glu-Leu-Asn-Arg-Tyr-Tyr-Ala-Ser-Leu-Arg-His-Tyr-Leu-Asn-Leu-Val-Thr-Arg-Gln-Arg-Tyr-NH₂ (peptide YY).

1.0 g (0.28 mmol) of Fmoc-Orn(4-methoxytrityl resin)-Tyr(tBu)-NH2 were coupled sequentially with Fmoc-Gln(Trt)-OH, Fmoc-Orn(Mmt)-OH, Fmoc-Thr(tBu)-OH, Fmoc-Val-OH, Fmoc-Leu-OH, Fmoc-Asn(Trt)-OH, Fmoc-Leu-OH, Fmoc-Tyr(tBu)-OH, Fmoc-His(Trt)-OH, Fmoc-Orn(Mmt)-OH, Fmoc-Leu-OH, Fmoc-Ser(tBu)-OH, Fmoc-Ala-OH, Fmoc-Tyr(tBu)-OH, Fmoc-Tyr(tBu)-OH, Fmoc-Orn(Mmt)-OH, Fmoc-Asn(Trt)-OH, Fmoc-Leu-OH, Fmoc-Glu(tBu)-OH, Fmoc-Glu(tBu)-OH, Fmoc-Pro-OH, Fmoc-Ser(tBu)-OH, Fmoc-Ala-OH, Fmoc-Asp(tBu)-OH, Fmoc-Glu(tBu)-OH, Fmoc-Gly-OH, Fmoc-Pro-OH, Fmoc-Ala-OH, Fmoc-Glu(tBu)-OH, Fmoc-Pro-OH, Fmoc-Lys(Boc)-OH, Fmoc-Ile-OH, Fmoc-Pro-OH and Fmoc-Tyr(tBu)-OH. The peptide was then cleaved from the resin and deprotected partially at the ornithine side chain and guanylated subsequently with 442.5 mg (3 mmol) of 1-H-1,2,4-triazole-carboxamidine hydrochloride in DMF and DIPEA. The product was then precipitated by the addition of water filtered, washed with water and DEE, deprotected, purified by RP-HPLC and lyophilized. Yield 289.9 mg (24.4%).

Various modifications and variations of the described aspects of the invention will be apparent to those skilled in the art. Although the invention has been described in connection with specific preferred embodiments, it should be understood that the invention as claimed should not be unduly limited to such specific embodiments. Indeed, various modifications of the described modes of carrying out the invention which are obvious to those skilled in the relevant fields are intended to be within the scope of the following claims.

## Claims

1. A method for preparing a peptide, or a salt thereof, from a peptide-resin conjugate of Formula (2b), (2c), (2d), (2e) or (2f), 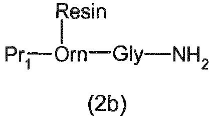 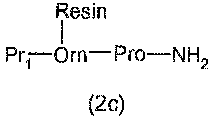 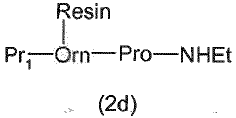 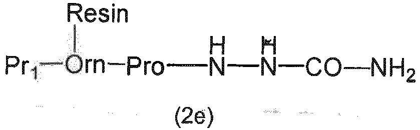 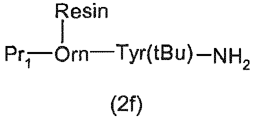
wherein the Resin is a TFA cleavable polymer resin selected from trityl, 2-chloro-trityl, 4-methyl-trityl and 4-methoxy-trityl resins; and
Pr₁ is a protecting group selected from the group consisting of Fmoc, Boc, Cbz, Npys and Alloc;
said method comprising the steps of:
(a) deprotecting the N-terminal α-amino function of said peptide-resin conjugate of Formula (2b), (2c), (2d), (2e) or (2f) to remove protecting group Pr₁;
(b) coupling an at least N-terminally protected amino acid or peptide having a free or activated carboxylic acid function with the deprotected α-amino function of step (a), thereby elongating the compound of formula (2b), (2c), (2d), (2e) or (2f),
(c) optionally repeating steps (a) and (b) one or more times, wherein the at least N-terminally protected amino acid or peptide is identical or different to that of the preceding step (b);
(d) cleaving the resulting peptide from the Resin;
(e) guanylating the peptide obtained in step (d);
(f) optionally removing all protecting groups which remain after step (d);
(g) isolating and optionally purifying the peptide thus obtained.

2. The method of claim 1, wherein the N-terminally protected amino acids or peptides of steps (b) and (c) are Fmoc-protected.

3. The method of claim 2, wherein the at least N-terminally protected amino acid or peptide of the lastly repeated step (c) is protected by an protecting group which is orthogonal to Fmoc.

4. The method of any one of claims 1 to 3, wherein step (d) comprises cleaving the peptide from the resin by treatment with an acid.

5. The method of any one of claims 1 to 4, wherein step (e) comprises treating the peptide with a guanylating reagent selected from 1H-pyrazole-1-carboxamidine 2, 1-H-1,2,4-triazole-carboxamidine, triflyl guanidine and benzotriazole-1-carboxamidinium tosylate.

6. The method of any one of claims 1 to 5, wherein the peptide resin-conjugate is selected from the following: 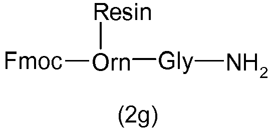 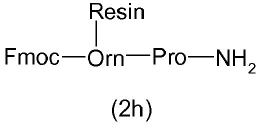 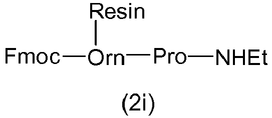 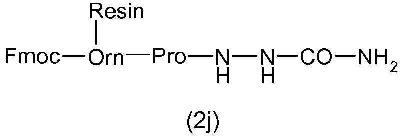 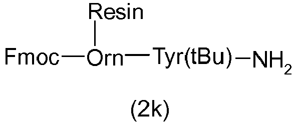 wherein the Resin is trityl resin or 4-methoxytrityl resin.

7. The method of claim 1 for preparing a peptide selected from the following:
[2] Arg-Cys-Tyr-Phe-Gln-Asn-Cys-Pro-Arg-Gly-NH₂;
[5] 3-Mercaptopropionyl-Tyr-Phe-Gln-Asn-Cys-Pro-D-Arg-Gly-NH₂;
[8] Pyr-His-Trp-Ser-Tyr-D-Ser(tBu)-Leu-Arg-Pro-Azagly-NH₂;
[9] Pyr-His-Trp-Ser-Tyr-D-Ser(tBu)-Leu-Arg-Pro-NHEt acetate salt;
[10] Pyr-His-Trp-Ser-Tyr-D-Leu-Leu-Arg-Pro-NHEt;
[11] Pyr-His-Trp-Ser-Tyr-D-His(Bzl)-Leu-Arg-Pro-NHEt acetate salt;
[12] Pyr-His-Trp-Ser-Tyr-D-Trp-Leu-Arg-Pro-NHEt acetate salt;
[20] 
H-Tyr-Pro-Ser-Lys-Pro-Asp-Asn-Pro-Gly-Glu-Asp-Ala-Pro-Ala-Glu-Asp-Met-Ala-Arg-
Tyr-Tyr-Ser-Ala-Leu-Arg-His-Tyr-Ile-Asn-Leu-Ile-Thr-Arg-Gln-Arg-Tyr-NH2; and
[21]

H-Tyr-Pro-Ile-Lys-Pro-Glu-Ala-Pro-Gly-Glu-Asp-Ala-Ser-Pro-Glu-Glu-Leu-Asn-Arg-
Tyr-Tyr-Ala-Ser-Leu-Arg-His-Tyr-Leu-Asn-Leu-Val-Thr-Arg-Gln-Arg-Tyr-NH2.

## Patentansprüche

1. Verfahren zur Herstellung eines Peptids oder eines Salzes davon aus einem Peptid-Harz-Konjugat der Formel (2b), (2c), (2d), (2e) oder (2f) 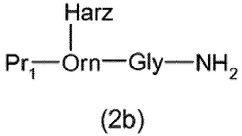 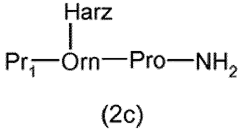 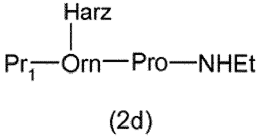 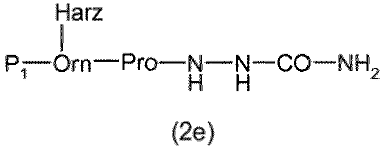 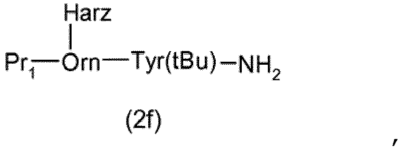
wobei es sich bei dem Harz um ein aus Trityl-, 2-Chlortrityl-, 4-Methyltrityl- und 4-Methoxytritylharzen ausgewähltes, durch TFA spaltbares Polymerharz handelt und
Pr₁ für eine aus der aus Fmoc, Boc, Cbz, Npys und Alloc bestehenden Gruppe ausgewählte Schutzgruppe handelt, wobei das Verfahren die folgenden Schritte umfasst:
(a) das Entschützen der N-terminalen α-Aminofunktion des Peptid-Harz-Konjugats der Formel (2b), (2c), (2d), (2e) oder (2f) zum Entfernen der Schutzgruppe Pr₁,
(b) die Kupplung einer mindestens N-terminal geschützten Aminosäure oder eines mindestens N-terminal geschützten Peptids mit einer freien oder aktivierten Carbonsäurefunktion mit der entschützten α-Aminofunktion von Schritt (a), unter Verlängerung der Verbindung der Formel (2b), (2c), (2d), (2e) oder (2f),
(c) gegebenenfalls das ein- oder mehrfache Wiederholen der Schritte (a) und (b), wobei die mindestens N-terminal geschützte Aminosäure bzw. das mindestens N-terminal geschützte Peptid gleich oder verschieden von dem aus dem vorhergehenden Schritt (b) ist,
(d) das Abspalten des erhaltenen Peptids vom Harz,
(e) das Guanylieren des in Schritt (d) erhaltenen Peptids,
(f) gegebenenfalls das Entfernen aller nach Schritt (d) verbliebenen Schutzgruppen,
(g) das Isolieren und gegebenenfalls Aufreinigen des so erhaltenen Peptids.

2. Verfahren nach Anspruch 1, wobei die N-terminal geschützten Aminosäuren oder Peptide von Schritt (b) und (c) Fmoc-geschützt sind.

3. Verfahren nach Anspruch 2, wobei die mindestens N-terminal geschützte Aminosäure oder das mindestens N-terminal geschützte Peptid des letzten Wiederholungsschritts (c) durch eine Schutzgruppe geschützt wird, die orthogonal zu Fmoc ist.

4. Verfahren nach einem der Ansprüche 1 bis 3, wobei Schritt (d) das Abspalten des Peptids vom Harz durch Behandlung mit einer Säure umfasst.

5. Verfahren nach einem der Ansprüche 1 bis 4, wobei Schritt (e) das Behandeln des Peptids mit einem Guanylierungsreagens ausgewählt aus 1H-Pyrazol-1-carboxamidin 2, 1-H-1,2,4-Triazolcarboxamidin, Triflylguanidin und Benzotriazol-1-carboxamidiniumtosylat umfasst.

6. Verfahren nach einem der Ansprüche 1 bis 5, wobei das Peptid-Harz-Konjugat aus den Folgenden ausgewählt ist: 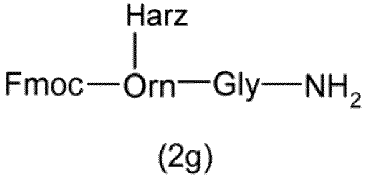 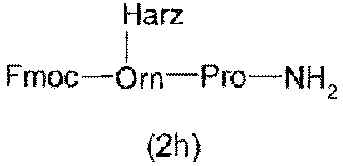 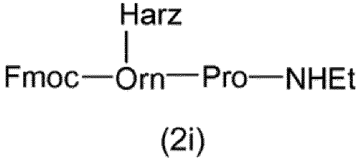 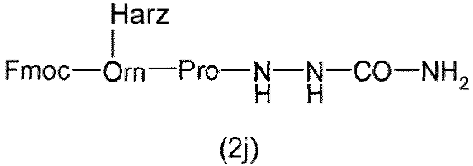 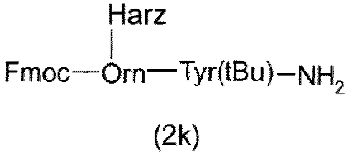 wobei es sich bei dem Harz um Tritylharz oder 4-Methoxytritylharz handelt.

7. Verfahren nach Anspruch 1 zur Herstellung eines Peptids ausgewählt aus den Folgenden:
[2] Arg-Cys-Tyr-Phe-Gln-Asn-Cys-Pro-Arg-Gly-NH₂,
[5] 3-Mercaptopropionyl-Tyr-Phe-Gln-Asn-Cys-Pro-D-Arg-Gly-NH₂,
[8] 
Pyr-His-Trp-Ser-Tyr-D-Ser(tBu)-Leu-Arg-Pro-Azagly-
NH2,
[9] 
Pyr-His-Trp-Ser-Tyr-D-Ser(tBu)-Leu-Arg-Pro-NHEt-
Acetatsalz,
[10] Pyr-His-Trp-Ser-Tyr-D-Leu-Leu-Arg-Pro-NHEt,
[11] 
Pyr-His-Trp-Ser-Tyr-D-His(Bzl)-Leu-Arg-Pro-NHEt-
Acetatsalz,
[12] 
Pyr-His-Trp-Ser-Tyr-D-Trp-Leu-Arg-Pro-NHEt-
Acetatsalz,
[20] 
H-Tyr-Pro-Ser-Lys-Pro-Asp-Asn-Pro-Gly-Glu-Asp-Ala-
Pro-Ala-Glu-Asp-Met-Ala-Arg-Tyr-Tyr-Ser-Ala-Leu-
Arg-His-Tyr-Ile-Asn-Leu-Ile-Thr-Arg-Gln-Arg-Tyr-NH2
 und
[21]

H-Tyr-Pro-Ile-Lys-Pro-Glu-Ala-Pro-Gly-Glu-Asp-Ala-
Ser-Pro-Glu-Glu-Leu-Asn-Arg-Tyr-Tyr-Ala-Ser-Leu-
Arg-His-Tyr-Leu-Asn-Leu-Val-Thr-Arg-Gln-Arg-Tyr-
NH2.

## Revendications

1. Procédé de préparation d'un peptide, ou d'un sel de celui-ci, à partir d'un conjugué peptide-résine de formule (2b), (2c), (2d), (2e) ou (2f), 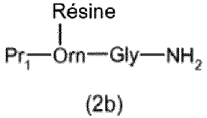 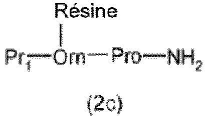 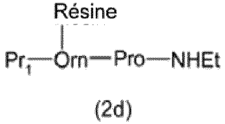 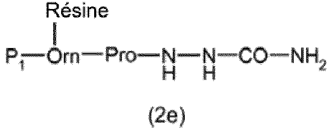 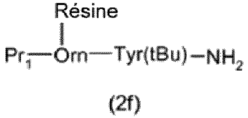
dans lequel la résine est une résine de polymère clivable par TFA choisie parmi des résines trityle, 2-chloro-trityle, 4-méthyl-trityle et 4-méthoxy-trityle ; et
Pr₁ est un groupe protecteur choisi dans le groupe constitué de Fmoc, Boc, Cbz, Npys et Alloc ;
ledit procédé comprenant les étapes de :
(a) déprotection de la fonction α-amino N-terminale dudit conjugué peptide-résine de formule (2b), (2c), (2d), (2e) ou (2f) de façon à éliminer le groupe protecteur Pr₁ ;
(b) couplage d'au moins un acide aminé ou peptide à protection N-terminale ayant une fonction acide carboxylique libre ou activée avec la fonction α-amino déprotégée de l'étape (a), de façon à allonger le composé de formule (2b), (2c), (2d), (2e) ou (2f),
(c) facultativement, répétition des étapes (a) et (b) une ou plusieurs fois, dans lequel l'au moins un acide aminé ou peptide à protection N-terminale est identique ou différent de celui de l'étape (b) précédente ;
(d) clivage du peptide résultant à partir de la résine ;
(e) guanylation du peptide obtenu dans l'étape (d) ;
(f) facultativement, élimination de tous les groupes protecteurs restants après l'étape (d) ;
(g) isolement et, facultativement, purification du peptide obtenu ainsi.

2. Procédé selon la revendication 1, dans lequel les acides aminés ou peptides à protection N-terminale des étapes (b) et (c) sont protégés par Fmoc.

3. Procédé selon la revendication 2, dans lequel l'au moins un acide aminé ou peptide de l'étape répétée en dernier (c) est protégé par un groupe protecteur qui est orthogonal à Fmoc.

4. Procédé selon l'une quelconque des revendications 1 à 3, dans lequel l'étape (d) comprend le clivage du peptide à partir de la résine par traitement avec un acide.

5. Procédé selon l'une quelconque des revendications 1 à 4, dans lequel l'étape (e) comprend le traitement du peptide avec un réactif d'agent de guanylation choisi parmi la 1H-pyrazole-1-carboxamidine 2, la 1H-1,2,4-triazole-carboxamidine, la triflyl-guanidine et le tosylate de benzotriazole-1-carboxamidinium.

6. Procédé selon l'une quelconque des revendications 1 à 5, dans lequel le conjugué peptide-résine est choisi parmi les suivants : 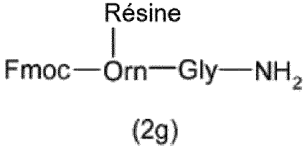 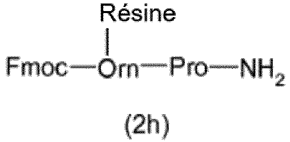 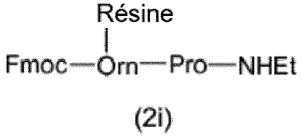 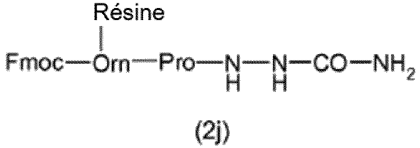 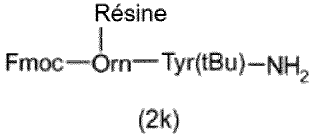 dans lequel la résine est une résine trityle ou une résine 4-méthoxytrityle.

7. Procédé selon la revendication 1 pour préparer un peptide choisi parmi les suivants :
[2] Arg-Cys-Tyr-Phe-Gln-Asn-Cys-Pro-Arg-Gly-NH₂ ;
[5] 3-mercaptopropionyl-Tyr-Phe-Gln-Asn-Cys-Pro-D-Arg-Gly-NH₂;
[8] Pyr-His-Trp-Ser-Tyr-D-Ser(tBu)-Leu-Arg-Pro-Azagly-NH₂;
[9] Pyr-His-Trp-Ser-Tyr-D-Ser(tBu)-Leu-Arg-Pro-NHEt, sel d'acétate;
[10] Pyr-His-Trp-Ser-Tyr-D-Leu-Leu-Arg-Pro-NHEt;
[11] Pyr-His-Trp-Ser-Tyr-D-His(Bzl)- Leu-Arg-Pro-NHEt, sel d'acétate ;
[12] Pyr-His-Trp-Ser-Tyr-D-Trp-Leu-Arg-Pro-NHEt, sel d'acétate ;
[20] H-Tyr-Pro-Ser-Lys-Pro-Asp-Asn-Pro-Gly-Glu-Asp-Ala-Pro-Ala-Glu-Asp-Met-Ala-Arg-Tyr-Tyr-Ser-Ala-Leu-Arg-His-Tyr-Ile-Asn-Leu-Ile-Thr-Arg-Gln-Arg-Tyr-NH₂; et
[21] H-Tyr-Pro-Ile-Lys-Pro-Glu-Ala-Pro-Gly-Glu-Asp-Ala-Ser-Pro-Glu-Glu-Leu-Asn-Arg-Tyr-Tyr-Ala-Ser-Leu-Arg-His-Tyr-Leu-Asn-Leu-Val-Thr-Arg-Gln-Arg-Tyr-NH₂.
